Europäisches Patentamt

European Patent Office

Office européen des brevets

(11) Veröffentlichungsnummer: **0 356 384**
**A2**

(12)

# EUROPÄISCHE PATENTANMELDUNG

(21) Anmeldenummer: **89810581.2**

(22) Anmeldetag: **28.07.89**

(51) Int. Cl.⁵: **C 01 B 17/69**
**C 12 P 3/00**

(30) Priorität: **11.08.88 CH 3031/88**

(43) Veröffentlichungstag der Anmeldung:
**28.02.90 Patentblatt 90/09**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Stucki, Gerhard, Dr.**
**Egghof**
**CH-4466 Ormalingen (CH)**

**Hüper, Heinz**
**Auf Käppelimatt 23**
**CH-4133 Prattein (CH)**

(54) **Wiederverwertung von Abfallschwefelsäure mit Hilfe biologischer Verfahren.**

(57) Gegenstand der vorliegenden Erfindung ist ein Recycling-Verfahren für Abfallschwefelsäure, dessen Kernstück die biologische Sulfat-Reduktion darstellt, wobei die Abfallschwefelsäure ohne Neutralisation unter Oxidation von organischen Verbindungen mit Hilfe mikrobiologischer Verfahren zu Sulfid reduziert und anschliessend über mehrere chemische Verfahrensstufen zu wiederverwertbarer Schwefelsäure aufgearbeitet wird.

**EP 0 356 384 A2**

**Beschreibung**

## Wiederverwertung von Abfallschwefelsäure mit Hilfe biologischer Verfahren

Gegenstand der vorliegenden Erfindung ist ein Recycling-Verfahren für Abfallschwefelsäure, dessen Kernstück die biologische Sulfat-Reduktion darstellt, wobei die Abfallschwefelsäure zunächst mit Hilfe mikrobiologischer Verfahren zu Sulfid reduziert und anschliessend über mehrere chemische sowie chemisch/physikalische Verfahrensstufen zu wiederverwertbaren Rohmaterialien, insbesondere zu wiederverwertbarer Schwefelsäure aufgearbeitet wird.

Schwefelsäure hat als Verbrauchsartikel der chemischen Industrie grosse Bedeutung und wird heute weltweit mit steigenden Produktionszahlen hergestellt. Verwendung findet die Schwefelsäure in erster Linie in der Agro-Industrie zur Herstellung von Phosphorsäure, Ammonium-Phosphaten und -Sulphaten (Düngemittelproduktion) sowie in der organisch-chemischen und petrochemischen Industrie als Hilfsmittel bei zahlreichen Synthesen, bei der Herstellung von Kunst- und Farbstoffen sowie beim Raffinieren von Erdöldestillaten.

Weitere Abnehmer von Schwefelsäure sind die Sprengstoff-, Waschmittel-, Lebensmittel-, Textil- und Papierindustrie.

Da jedoch Schwefelsäure in der Hauptsache als Reaktionsmittel oder Hilfsmittel und nicht als Reaktionspartner eingesetzt wird, fallen in der Regel grosse Mengen an unterschiedlich konzentrierter, verunreinigter und gebrauchter Abfallschwefelsäure an.

Die umweltgerechte und kostengünstige Entsorgung dieser verunreinigten und für die weitere Verwendung ungeeigneten Abfallschwefelsäure stellt nach wie vor ein grosses Problem dar. Eines der heute noch vielfach verwendeten Verfahren besteht in der Neutralisation der verunreinigten Säure mit Kalk und der Ablagerung des anfallenden Gips in Gips-Deponien. Aufgrund der damit verbundenen Umweltrisiken (Grundwasserbelastung etc.) und der immer knapper werdenden Deponiekapazitäten ist es eine vordringliche Aufgabe neue, umweltschonende und nach Möglichkeit kostengünstige Alternativen für die Beseitigung der Abfallschwefelsäure zu entwickeln.

Schwach belastete Schwefelsäuren werden daher heute z.B. zu Neutralisationszwecken in Kläranlagen eingesetzt oder werden, nach vorangegangener Neutralisation, über die Abwasserreinigungsanlagen entsorgt.

Eine vielversprechende Alternative zur Entsorgung bilden Verfahren, die eine Wiederverwertung von Abfallschwefelsäure ermöglichen.

Solche Verfahren sind bereits bekannt, wie z.B. das destillative Recycling-Verfahren, das thermische Spaltverfahren, die thermische Spaltung nach dem Grillo Prozess und andere.

Die genannten Verfahren haben jedoch alle gravierende Nachteile, wobei in erster Linie hohe Investitions- und Prozesskosten zu nennen sind, die eine grosstechnische Anwendung aus ökonomischer Sicht wenig attraktiv erscheinen lassen. Darüberhinaus sind die oben erwähnten Verfahren nur bei Verwendung konzentrierter (> 20 %) und nur mässig verunreinigter Schwefelsäuren anwendbar.

Weitere Verfahren zur Wiedergewinnung von Sulfat-Schwefel, die zur Zeit diskutiert werden, basieren auf biologischen Verfahrensmassnahmen. Es ist nämlich seit langem bekannt, dass Sulfat-reduzierende Bakterien (SRB) in der Lage sind, unter anaeroben Bedingungen bereits geringste Mengen von Sulfat zu $H_2S$ zu reduzieren. Die für ihr Wachstum benötigte Energie beziehen die SRB dabei von (meist organischen) Wasserstoff-(Elektronen-)Donoren.

Der auf diese Weise gebildete Schwefelwasserstoff lässt sich mit bekannten chemischen Prozessen oder biologischen Methoden oxidieren. Mögliche Produkte sind Schwefel, Sulfide oder Hydrogensulfide. Mit geeigneten Methoden zur Oxidation von $H_2S$ aus der Sulfat-Reduktion lässt sich eine nahezu komplette Wiederverwertung im Bezug auf die Schwefelbilanz realisieren.

Ein biologisches Recycling von Sulfat-Schwefel ist im Prinzip möglich, wie aus zahlreichen Publikationen hervorgeht [ [1])Burgess S.G. and Wood L.B., J.Sci.Food Agric., 12: 326-335, 1961; [2])Ueki et al., Gen.Appl.Microbiol., 27: 457-464, 1981]. Trotz den Arbeiten von Cork und Mitarbeitern [ [3])Cork D. and Cusanovich M., Dev.Ind.Microbiol., 20: 591-602, 1979; [4])Cork D., Dev.Ind.Microbiol., 23: 379-387, 1982, [5])Cork D. et al., Appl.Env.Microbiol., 45: 913-918, 1983, [6])Cork D., DE-OS 33 28 500 A1, [7])Cork D. et al., Appl.Env.Microbiol., 49(2): 269-272, 1985, [8])Cork D. et al., Biotech. and Bioeng.Sym. No. 16: 149-162, 1986, [9])Grimm D. et al., Dev.Ind.Microbiol., 25: 709-716, 1984), [10])Olthof (Olthof M. et al., DE-OS 34 22 959 A1), Maree und Strydom ( [11])Maree J.P. and Strydom W.F., Water Res., 19(9): 1101-1106, 1985) sowie [11a]) Mulder A. (EP-A 241,999)], die nähere Angaben über Versuchsbedingungen und Apparaturen veröffentlichen, ist die Sulfat-Reduktion als Variante zu bisherigen Methoden zur Entsorgung von Abfall-Schwefelsäure nicht konkurrenzfähig, da die bisher erzielten Umsatzraten für eine grosstechnische Nutzung des Verfahrens zu gering sind.

Eine vordringliche Aufgabe bestand daher darin, ein Verfahren zu entwickeln, das eine deutliche Erhöhung der Sulfat-Umsatzraten ermöglicht.

Diese Aufgabe konnte jetzt im Rahmen der vorliegenden Erfindung überraschenderweise durch die Anwendung verbesserter Verfahrensmassnahmen gelöst werden, wobei die hier erzielten Sulfat-Umsatzraten wenigstens doppelt so hoch liegen wie die bisher publizierten Werte.

Darüberhinaus konnte mit Hilfe des erfindungsgemässen Verfahrens durch den Einsatz billiger Energiequellen die Kostenbilanz deutlich verbessert werden, sodass nunmehr eine unter ökonomischem und umweltpolitischem Gesichtspunkt interessante Verfahrensvariante für die Wiederverwertung von Abfallschwefelsäure zur Verfügung steht.

Die vorliegende Erfindung betrifft ein Dreistufen-Verfahren für das Recycling von Abfallschwefelsäure, das dadurch gekennzeichnet ist, dass man Abfallschwefelsäure ohne vorhergehende Neutralisation

a) in einer ersten, rein biologischen Verfahrensstufe mit Hilfe Sulfat-reduzierender Mikroorganismen unter strikt anaeroben Bedingungen zu Sulfid reduziert,

b) das für die Sulfat-reduzierenden Mikroorganismen toxische Sulfid durch Zugabe von Metall-Salzen aus dem Inkubationsmedium entfernt und

c) das gefällte Metallsulfid in einer dritten Verfahrensstufe mit ausschliesslich chemisch/physikalischen Methoden zu wiederverwertbaren Rohmaterialien, wie z.B. wiederverwertbarem Sulfid oder wiederverwertbarer Schwefelsäure aufarbeitet.

Aufgrund der erfindungsgemässen Prozessführung ist es nunmehr möglich, sehr hohe Umsatzraten für die Sulfat-Reduktion zu erzielen, die die heute bekannten und erreichbaren Umsatzraten deutlich übertreffen.

Dies wird erfindungsgemäss unter anderem dadurch erreicht, dass der für die Sulfat-reduzierenden Mikroorganismen toxische Schwefelwasserstoff, der im Zuge der biologischen Sulfat-Reduktion gebildet wird, durch Zugabe von Metall-Salzen schnell und effizient aus dem Bio-System entfernt wird. Dabei ist es gelungen, durch Einbeziehen einer Trenneinheit in das erfindungsgemässe Verfahren, eine Anreicherung von prezipitiertem Metall-Sulfid im Bioreaktor zu vermeiden und die damit verbundenen negativen Einflüsse auf die Konzentration und die Aktivität der Biomasse zu verhindern. Das in Form von Metallsulfid ausgefällte Sulfid kann entweder zwischengelagert oder in einer dritten Verfahrensstufe direkt zu Schwefelsäure weiterverarbeitet werden. Das dabei freigesetzte Metall-Salz wird wieder in den Prozesskreislauf zurückgeführt.

Im einzelnen besteht das erfindungsgemässe Verfahren aus den folgenden Verfahrensmassnahmen

1) Reduktion von Sulfat aus Abfallschwefelsäure durch Sulfat-reduzierende Mikroorganismen zu $H_2S$;

2) Kontinuierliches Entfernen von toxischem $H_2S$ aus dem Bio-Reaktor-Medium durch dosierte Zugabe von Metallsulfat, das mit dem anfallenden Sulfid zu Metallsulfid (MeS) reagiert.

3) Abtrennen des ausgefällten Metallsulfids mit Hilfe an sich bekannter Trennverfahren.

4) Versetzen der entfernten MeS-Suspension mit Abfallschwefelsäure unter Freisetzung hochkonzentrierten $H_2S$-Gases.

5) Direkte Aufoxidation von $H_2S$ zu $H_2SO_4$, und Zurückführen der in 4) entstehenden $MeSO_4$-Lösung in den Prozesskreislauf zum Ausfällen von Sulfid aus dem Bioreaktor-Medium und zur weiteren Zugabe von Sulfat als Substrat.

6) Versetzen der aus 2 und 3 resultierenden $H_2S$-armen, Sulfat-haltigen und gegebenenfalls von $CO_2$ befreiten Lösung mit einer C-Quelle sowie geeigneten Nährsalzen und Zurückführen in Verfahrensstufe 1.

Bei den in der 1. (biologischen) Verfahrensstufe eingesetzten Organismen handelt es sich vorzugsweise um Sulfat-reduzierende Mikroorganismen (SRM), die unter Ausschluss von Sauerstoff organische Verbindungen oxidieren und gleichzeitig Sulfat zu Schwefelwasserstoff reduzieren.

Insbesondere sind im Rahmen dieser Erfindung Sulfat-reduzierende Bakterien (SRB) geeignet, von denen schon seit langem bekannt ist, dass sie in der Lage sind unter anaeroben Bedingungen bereits kleinste Mengen an Sulfat zu Sulfid zu reduzieren.

Im Zuge dieser dissimilatorischen Sulfat-Reduktion dient Sulfat als terminaler Elektronenakzeptor. Dabei werden grosse Mengen an Sulfid in Form von Schwefelwasserstoff freigesetzt.

$$8[H+] + SO_4^{2-} \rightarrow H_2S + 2H_2O + 2OH^-.$$

Die SRB verwenden in der Regel organische Säuren, wie Lactat oder Acetat, Alkohole oder molekularen Wasserstoff als H-Donatoren. Die organischen Substrate werden dabei nicht immer endoxidiert, sodass oft Nebenprodukte anfallen. Viele Stämme verfügen über eine konstitutive Hydrogenase, die molekularen Wasserstoff in die Atmungskette einschleust und somit eine Chemolitothrophie ermöglicht.

Der Elektronentransfer vom organischen Substrat auf $SO_4^{2-}$ erfolgt über spezielle Elektronen-Carrier vom Cytochrom-Typ.

Die Gattung Desulfovibrio enthält beispielsweise Cytochrome vom $c_3$-Typ, während man bei Desulfotomaculum Cytochrome vom b-Typ findet. Am Elektronentransport sind daneben auch Eisenproteine, Flavoproteine und Chinone beteiligt, sodass die SRB aufgrund der vielen eisenhaltigen Proteine, die am Elektronentransport beteiligt sind, auf eine Fe-reiche Umgebung angewiesen sind.

Die Reduktion von Sulfat ist an eine Elektronentransport-Phosphorylierung gekoppelt und gleicht somit formal der Atmung.

Im Rahmen der vorliegenden Erfindung können je nach Verwendung der organischen Kohlenstoff- und Energie-Quelle verschiedene Mikroorganismen zum Einsatz gelangen, die zum Grossteil bekannt sind und bei verschiedenen Institutionen, wie z.B. bei der "Deutschen Sammlung von Mikroorganismen (DSM)", Göttingen, BRD, oder der "American Type Culture Collection (ATCC), 12301 Park Lawn Drive, Rockville, Maryland 20852, USA, bezogen werden können.

Beispielhaft seien im folgenden verschiedene Vertreter aus der Gruppe der Sulfat-reduzierenden Bakterien genannt, ohne dadurch jedoch den Erfindungsgegenstand in irgendeiner Weise zu limitieren:

Desulfovibrio, Desulfotomaculum, Desulfobacter, Desulfomonas, Desulfobulbus, Desulfococcus, Desulfonema, Desulfosarcina, Thermodesulfobacterium [ [12])Pfennig N et al, (1981) "The Dissimilatory Sulfate-reducing Bacteria", in: "The Prokaryotes" - A Handbook of Habitats, Isolation and Identification of Bacteria", Vol. 1 Springer Verlag, Berlin; [13])Trüper HG (1984) "Microorganisms and the Sulfur Cycle", in: Müller A and Krebs B, "Sulfur, its Significance for Chemistry, for the Geo-, Bio- and Cosmosphere and Technology", Vol. No. 5, Elsevier, Amsterdam; [14])Widdel F (1980), "Anaerober Abbau von Fettsäuren und Benzolsäure durch neu isolierte Arten Sulfat-reduzierender Bakterien, Dissertation, Universität Göttingen, BRD].

Ganz besonders geeignet für die Verwendung in dem erfindungsgemässen Verfahren sind Desulfobacter postgatei (DSM 2034, DSM 2553), Desulfotomaculum acetoxidans (DSM 771) sowie Desulfotomaculum orientis (DSM 765).

Diese Sulfat-reduzierenden Bakterienstämme können entweder einzeln oder aber bevorzugterweise in Kombination in dem erfindungsgemässen Verfahren eingesetzt werden. Die Anzucht der Stämme erfolgt gemäss den Angaben der Hinterlegungsstelle (Deutsche Sammlung von Mikroorganismen in Göttingen, BRD).

In einer spezifischen Ausführungsform der vorliegenden Erfindung werden für die Umsetzung der Abfallschwefelsäure Mischkulturen Sulfat-reduzierender Mikroorganismen verwendet, die in Form von Anreicherungskulturen aus natürlichen Habitaten besagter Organismen isoliert werden können.

Sulfat-reduzierende Bakterien sind in anaeroben aquatischen oder terrestrischen Systemen weitverbreitet [ [15])Pfennig N and Biebl H, Arch.Microbiol., 110: 3-12, 1976]. Eine ganze Reihe dieser anaeroben Mikroorganismen konnten aus den unterschiedlichsten anaeroben Lebensräumen isoliert werden, die einen Sulfat-Ueberschuss, sowie ein genügend grosses Angebot an Energiequellen aufweisen, wie zum Beispiel die Sedimente von Flussmündungen [ [16])Laanbrock H, et al., Arch.Microbiol., 133: 178-184, 1982), Meeren ( [17])Widdel F and Pfennig N, Arch.Microbiol., 129: 395-400, 1981; [18])Widdel F and Pfennig N, Arch.Microbiol., 131: 360-365, 1982), Watten und salzhaltiger Seen und Teiche ( [19])He S, Biochem.Biophys.Res.Comm., 135(3): 1000-1007, 1986].

Auch an Sulfat-limitierten Orten oder an Plätzen mit schwankendem Sulfat-Gehalt werden Desulfurikanten gefunden. Sie sind ubiquitär in Faulschlämmen von Kläranlagen [ [21])Badziong W and Thauer R, Arch.Microbiol., 117: 209-214, 1978; [15])Pfennig N and Biebl H, Arch.Microbiol. 110: 3-12, 1976; [21])Schlegel H G, Allgemeine Mikrobiologie, 6. Auflage, Thieme Verlag, Stuttgart, 1986; [2])Ueki et al., Gen.Appl.Microbiol., 27: 457-464, 1981] und kommen vor in Süsswasserflüssen und Teichen [ [22])Badziong W et al., Arch.Microbiol., 116: 41-49, 1978], Tropischen Seen [ [19])He S, Biochem.Biophys.Res.Comm., 135(3): 1000-1007, 1986], Grundwässern auf Oelfeldern [ [23])Antloga K M, and Griffin W M, Dev.Ind.Microbiol. 26: 597-610, 1985], Schweinemist und Torf-Sümpfen [ [24])Widdel F and Pfennig N, Arch.Microbiol., 112: 119-122, 1977]. Einige Spezialisten unter den Sulfat-Reduzenten sind an extreme Standorte angepasst, wie z.B. an heisse, vulkanische Quellen [Thermodesulfobacterium commune ($\mu_{max}$ bei 70°C)] [ [25])Zeikus J G et al., J.Gen.Microbiol., 129: 1159-1169, 1983], extrem salzhaltige Medien [Desulfovibrio salexigens (max. Salztoleranz: ca. 50 g NaCl/l)] sowie Mägen von Wiederkäuern [Desulfotomaculum ruminis] [ [12])Pfennig N et al, (1981) "The Dissimilatory Sulfate-reducing Bacteria", in: "The Prokaryotes" - A Handbook of Habitats, Isolation and Identification of Bacteria", Vol. 1 Springer Verlag, Berlin; [21])Schlegel H G, Allgemeine Mikrobiologie, 6. Auflage, Thieme Verlag, Stuttgart, 1986].

Besonders bevorzugt im Rahmen dieser Erfindung sind Isolate aus Faulschlämmen von Kläranlagen, die in der Regel eine Vielzahl Sulfat-reduzierender Mikroorganismen in bereits angereicherter Form enthalten. Durch die Wahl geeigneter Energie-, Kohlenstoff- und Stickstoffquellen, eines Wasserstoffakzeptors sowie verschiedener chemisch/physikalischer Parameter, wie Licht, Temperatur, pH-Wert, Redoxpotential u.a. ist es möglich ganz gezielt, die für die jeweilige Problemstellung am besten geeignete Mischpopoulation von SRB anzureichern. Diese Verfahren zur Anreicherung Sulfat-reduzierender Mikroorganismen sind dem Fachmann bekannt und können ohne unzumutbaren experimentellen Aufwand von diesem durchgeführt werden.

Zur Isolation und Anreicherung von SRB sind bereits eine Reihe von Medien in der Literatur beschrieben.

Diese sogenannten Starter-Medien enthalten neben einer leicht assimilierbaren C-Quelle wie z.B. Lactat, Propionat und Acetat vor allem die für die Erhaltung und Vermehrung der Mikroorganismen notwendigen mineralischen Stoffe bzw. Elemente in ihren Verbindungen, wie Stickstoff, Phosphor, Schwefel, Kalium, Natrium, Calcium, Magnesium und Eisen, neben sogenannten Spurenelementen, wie Zink, Kupfer, Mangan, Bor, Vanadium, Selen und Molybdenum.

Weitere Bestandteile besagter Starter-Medien für die Anreicherung Sulfat-reduzierender Bakterien umfassen Hefeextrakte und/oder Vitamine, wie Cyano-Cobalamin, Pyridoxin, Ca-Pantothenat, Riboflavin, Biotin, Liponsäure, Nicotinsäure, Folsäure und Thiamin.

Die Isolierung und Kultivierung der SRB muss unter strikt anaeroben Bedingungen durchgeführt werden. Man verwendet daher vorzugsweise Gefässe oder Bioreaktoren, die luftdicht abgeschlossen werden können.

Weiterhin ist es vorteilhaft unter einer künstlichen Gas-Atmosphäre zu arbeiten, wobei als Inertgase hochreiner, Sauerstoff-freier Stickstoff (> 99,999 %), $CO_2$, Argon [ [26])Robinson J A and Tiedje J M, Arch.Microbiol., 137: 26-32, 1984] oder Mischungen dieser Gase verwendet werden.

Um SRB in Reinkulturen zu isolieren, kann die Hungate-Technik verwendet werden [ [27])Hungate R E, "A Roll-Tube Method for Cultivation of strict Anaerobes", in: Norris, Ribbons, Methods in Microbiology, Vol. 3B, 117-132, Academic Press, N.Y., 1969]. Sie ist in mehreren Varianten erprobt und vielseitig in der Literatur beschrieben [ [28])Braun M and Stolp H, Arch.Microbiol., 142: 77-80, 1985; [26])Robinson J A and Tiedje J M, Arch.Microbiol., 137: 26-32, 1984; [2])Ueki et al., Gen.Appl.Microbiol., 27: 457-464, 1981]. Eine Kultivierung im Fermenter ist ebenfalls möglich [ [20])Badziong W and Thauer R, Arch.Microbiol., 117: 209-214, 1978].

Der pH-Wert des Starter-Mediums wird vorzugsweise auf einen Wert zwischen pH 7 und pH 8, insbesondere auf einen Wert zwischen pH 7,2 und 7,8 eingestellt.

Das Starter-Medium wird anschliessend für einen Zeitraum von 20-30 h bei einer Temperatur zwischen 20°C und 40°C mit aeroben und/oder fakultativ anaeroben Mikroorganismen vorinkubiert, die beispielsweise in Klärschlämmen oder an deren natürlichen Habitaten vorkommen.

Während dieser Zeit wird die vorgelegte C-Quelle unter Verbrauch des im Medium befindlichen Sauerstoffs von aeroben und/oder fakultativ anaeroben Mikroorganismen verwertet.

Mit Hilfe eines zuvor dem Medium zugesetzten Indikatorfarbstoffes, wie z.B. einer 0,2 % Resazurin-Lösung, kann der Zeitpunkt, an dem der gesamte Sauerstoff des Mediums verbraucht ist und somit anaerobe Bedingungen vorliegen, anhand des Farbumschlags exakt bestimmt werden.

Erst wenn strikt anaerobe Bedingungen vorliegen, wird das Starter-Medium zur Anreicherung der Sulfat-reduzierenden Bakterien mit dem die SRB enthaltenden Impfgut anaerob beimpft.

Nach einer Anlaufphase von 2-10 Tagen, die für eine Stabilisierung des Systems notwendig ist, wird der Bioreaktor kontinuierlich mit einem Medium beschickt, das, neben einer geeigneten C- und Energie-Quelle, den zuvor genannten essentiellen Mineralsalzen und Vitaminen, auch Sulfat aus Abfallschwefelsäure enthält. Die Zudosierung des "Abfallschwefelsäure-Mediums" erfolgt dabei nur sehr langsam mit einer Anfangsdosis von 1 bis 30 ml/l/Tag.

Es ist dabei vorteilhaft den Prozess so zu führen, dass die Salzkonzentration im Medium konstant bleibt, vorzugsweise in einem Bereich von 2g/l bis 30 g/l und ganz besonders bevorzugt in einem Bereich von 2g/l bis 10 g/l. Als C- und Energiequelle für die Sulfat-Reduktion verwendet man vorzugsweise von den SRB leicht verwertbare organische Verbindungen, insbesondere Propionat, Lactat, Isopropanol und Acetat. Die bevorzugte Konzentration im Bioreaktor liegt in einem Bereich von 0.1 g/l bis 25 g/l, vorzugsweise aber bei einer Konzentration von 0.3 g/l bis 3 g/l. In einer spezifischen Ausführungsform der vorliegenden Erfindung dient Methanol und/oder Acetat als C- und Energiequelle, wobei insbesondere ein Methanol/Acetat-Gemisch in einem Mischungsverhältnis von 1:1 bis 40:1 bevorzugt ist.

Ganz besonders bevorzugt im Rahmen dieser Erfindung ist die Verwendung von Acetat. Bei Verwendung höher konzentrierter Cabonsäuren, wie z.B. einer > 2%-igen Essigsäure, ist es vorteilhaft das entstehende $CO_2$ aus dem Kulturmedium zu entfernen, da dies den Prozessverlauf negativ beeinflussen kann.
Dies kann mit Hilfe bekannter Verfahren geschehen, wie z.B. durch $CO_2$-Stripping.

Die Sulfat-Reduktion wird zweckmässigerweise bei einer Prozess-Temperatur von 20° bis 50°C durchgeführt. Optimale Wachstumsvoraussetzungen finden die mesophilen Desulfurikanten insbesondere in einem Temperaturbereich von 30° bis 40°C, der im Rahmen dieser Erfindung daher besonders bevorzugt ist.

Bei Verwendung thermophiler Mikroorganismen können die Prozesstemperaturen dagegen bis auf Werte von >50°C gesteigert werden.

Der optimale pH-Bereich für die Sulfat-Reduktion liegt bei mittleren Werten zwischen pH 7.0 und pH 9.0, vorzugsweise zwischen pH 7.0 und pH 8.5, und ganz besonders bevorzugt bei mittleren Werten zwischen pH 7.2 und pH 7.8.

Die in dem erfindungsgemässen Verfahren eingesetzten Mikroorganismen können in Form grösserer Aggregate und Pellets, sowie insbesondere in immobilisierter Form vorliegen.

Anaerobe Mikroorganismen, zu denen auch die SRB gehören, zeichnen sich durch lange Generationszeiten aus (12 h bis 24 h). Dies ist dadurch bedingt, dass die biochemische Energie-Ausnützung beim Abbau der orga nischen Substanzen unter Ausschluss von Sauerstoff beschränkt ist. Die Stoffwechsel-Aktivität von Anaerobiern hingegen ist vergleichbar mit derjenigen von aeroben Bakterien.

Ein Problem bei der Sulfat-Reduktion liegt in der Erzielung einer hohen Raum-Zeit-Ausbeute, d.h. möglichst viel Sulfat in einem kleinen Reaktorvolumen und mit kurzer Verweildauer umzusetzen. Diese Forderung ist umso einfacher zu erfüllen, je höher die Konzentration der aktiven Biomasse im Reaktor ist. Wegen des Energie-limitierenden Wachstums werden aber z.B. in einem Rührkessel nur geringe Biomasse-Konzentrationen erreicht; zudem machen die langen Generationszeiten entsprechend lange Verweilzeiten in kontinuierlichen Systemen erforderlich.

Da die Mikroorganismen eine katalytische Rolle bei der Sulfat-Reduktion spielen, ist es notwendig, dass Verluste im Ablauf von Reaktoren vermieden werden. Dies kann mit verschiedenen Methoden, insbesondere mit einer Biomasse-Rückhaltung oder Biomasse-Rückführung, erreicht werden. Durch diese Verfahrensmassnahmen wird die Feststoff-Verweilzeit (Biomasse, Biokatalysator) von der Flüssigkeits-Verweilzeit entkoppelt. Die Mikroorganismen reichern sich im Reaktor an was letztlich zu einer Vervielfachung der Umsatzleistung der Sulfat-Reduktion führt.

Zur Rückführung anaerober Mikroorganismen sind verschiedene Methoden und Reaktortypen beschrieben. [ 29)Aivasidis und Wandrey Berichte der Kernforschungsanlage Jülich, Nr. 1900, Institut für Biotechnologie 2 Jül-1900, 1984; 30)Mc Carty und Smith PL and Smith DP, Environ.Sci.Technol. 20(12): 1200-1206, 1986; 31)Baumann G, Korrespondenz Abwasser, 1: 28.37, 1987].

Bevorzugt im Rahmen dieser Erfindung ist die Verwendung der SRB in immobilisierter Form, wodurch eine Teilchenvergrösserung erreicht wird, welche die Sedimentationseigenschaften der SRB deutlich verbessert und damit gleichzeitig die Abtrennbarkeit aus der flüssigen Phase entscheidend erleichtert.

Die Immobilisierung besagter SRB kann erfindungsgemäss auf verschiedene Art und Weise, analog zu an sich bekannten Verfahren, durchgeführt werden.

Im Rahmen der vorliegenden Erfindung sind in erster Linie Verfahren zu nennen, die auf einer adsorptiven, ionischen oder kovalenten Bindung besagter Biokatalysatoren an feste, in der Regel wasserunlösliche Trägermaterialien, auf einer Quervernetzung von Biokatalysatoren durch bi- oder mehrfunktionelle Reagentien, einer Matrixumhüllung, einer Membranabtrennung oder einer Kombination zweier oder mehrerer der oben genannten Verfahren beruhen.

Ebenfalls umfasst von dem breiten Konzept der vorliegenden Erfindung werden Verfahren, bei denen sich die Mikroorganismen aufgrund der gewählten Reaktorbedingungen selbst zu einem Verband immobilisieren und dadurch von der Flüssigkeitsverweilzeit entkoppelt werden ['granular sludge'; Lettinga G et al., Biotechnology and Bioengineering, Vol. XXII, pp 699-734, John Wiley & Sons, Inc. 1980].

Die adsorptive Bindung an wasserunlösliche Trägerstoffe (Adsorbentien) erfolgt in erster Linie aufgrund von van der Waal-Wechselwirkungskräften. Als Adsorbentien kommen zahlreiche anorganische und organische Verbindungen, desgleichen auch synthetische Polymere in Betracht.

Methoden für eine derartige Immobilisierung von Mikroorganismen sind bei [32]van Haecht et al, 1985 (Hefezellen/Glas), [33]Black et al, 1984 (Hefezellen/Edelstahl, Polyester), [34]Wiegel und Dykstra, 1984 (Clostridien/Cellulose, Hemicellulose), [35]Förberg und Häggström, 1984 (Clostridien/Holzspäne), [36]Ehrhardt und Rehm, 1985 (Pseudomonaden/Aktivkohle) sowie bei [29]Aivasidis und Wandrey, 1984 (poröses Sinterglas) beschrieben.

Ionische Bindungen basieren auf elektrostatischen Anziehungen zwischen entgegengesetzt geladenen Gruppen des Trägermaterials (wie beispielsweise handelsüblichen Ionenaustauschern z.B. auf der Basis von Polysacchariden oder von synthetischen Harzen) und des zu bindenden Biokatalysators.

Auf ionischer Bindung basierende Methoden zur Immobilisierung von Mikroorganismen sind bei [37]DiLuccio und Kirwan, 1984 (Azotobacter spec./Cellex E (Cellulose)) sowie bei [38]Giard et al., 1977 (Tierische Zellen/DEAE-Sephadex) beschrieben.

Eine weitere Methode zur Immobilisierung beruht auf der Ausnützung von kovalenten Bindungskräften, die im allgemeinen zu festen Verknüpfungen von Biokatalysatoren untereinander oder zwischen Biokatalysator und Trägermaterial führen, wobei als Trägermaterialien, poröse Materialien, wie Gläser, Kieselerde oder andere, unlösliche, anorganische Materialien eingesetzt werden können.

Im Rahmen des erfindungsgemässen Verfahrens kann man die Mikroorganismen z.B. analog zu [39]Messing und Oppermann, 1979 (Enterobakterien/Borosilikatglas; Hefezellen/Zirkonerde), [40]Romanovskaya et al., 1981 (Methanbakterien/Silochrome), [41]Navarro und Durand, 1977 (Hefezellen/poröse Kieselerde) immobilisieren.

Im erfindungsgemässen Verfahren kommen nicht nur die bereits genannten Trägermaterialien für eine Immobilisierung in Betracht sondern eine ganze Reihe von natürlichen oder synthetischen Polymeren, wie z.B. Cellulose, Dextran, Stärke, Agarose usw. oder Polymere z.B. auf der Basis von Acryl-und Methacrylsäurederivaten, die üblicherweise bei der Herstellung reaktiver Copolymerisate Verwendung findet. Als reaktive Gruppen über die eine Bindung mit dem Biokatalysator vermittelt wird sind reaktionsfähige Dinitrofluorphenyl- oder Isothiocyanatgruppen vor allem Oxiran- und Säureanhydridgruppen zu nennen. Eine weitere Möglichkeit besteht in der Chloridaktivierung von Carboxyl-Gruppen tragenden Harzen, die beispielsweise unter den Handelsnamen Amberlite XE-64 und Amberlite IRC-50 kommerziell erhältlich sind.

Die Immobilisierung von Mikroorganismen mit Hilfe von natürlichen oder synthetischen Trägermaterialien kann wie bei [42]Chipley, 1974 (Bacillus subtilis/Agarose), [43]Gainer et al., 1980 (Azotobacter species/Cellulose), [44]Jack and Zajic, 1977 (Micrococcus luteus/Carboxymethylcellu lose), [45]Jirku et al., 1980 (Hefezellen/Hydroxyalkylmethacrylat) sowie bei [46]Shimizu et al., 1975 (Bakterienzellen/Ethylen-Maleinanhydrid-Copolymer) beschrieben, durchgeführt werden.

Bei der Quervernetzung ("cross linking") werden die Biokatalysatoren durch bi- oder mehrfunktionelle Reagentien, wie Glutardialdehyd, Diisocyanate u.a., miteinander verbunden und bilden hochmolekulare, typischerweise unlösliche, meist gelartige Aggregate.

Derartige Immobilisierungen von Mikroorganismen kann man analog zu [47]De Rosa et al., 1981 (Bakterienzellen/Co-Crosslinking mit Hühnereiweiss durch Glutardialdehyd) durchführen.

Die Matrixumhüllung beinhaltet den Einschluss der Biokatalysatoren in natürliche oder synthetische Polymere, die meist gelartige Struktur aufweisen. Besonders geeignet als Matrixmaterialien für den Einschluss von Zellen, Organellen und Sporen sind natürliche Polymere wie Alginat, Carrageenan, Pectin, Agar, Agarose oder Gelatine, da diese Verbindungen nicht toxisch und in ihrer Handhabung zellschonend sind.

Weiterhin kommen in Frage synthetische Polymere, wie beispielsweise Polyacrylamide, photovernetzte Harze u.a. Die Gestaltung der Matrixumhüllungen ist innerhalb weiter Grenzen variabel und kann beispielsweise Kugel-, Zylinder-, Faser- oder Foliengestalt umfassen.

Die Immobilisierung von Mikroorganismen mit Hilfe von natürlichen oder synthetischen Matrixmaterialien kann wie bei [48]Mazumder et al., 1985 (Bakterienzellen/photovernetzte Harze), [49]Bettmann und Rehm, 1984 (Bakterienzellen/Polyacrylamidhydrazid), [50]Umemura et al., 1984 (Bakterienzellen/Carrageenan), [51]Karube et al., 1985 (Bakterienprotoplasten/Agar-Acetylcellulose), [52]Cantarella et al., 1984 (Hefezellen/Hydroxyethylmethacrylat), [53]Qureshi und Tamhane, 1985 (Hefezellen/Alginat), [54]Deo und Gaucher, 1984 (Schimmelpilzmycel/Carrageenan), [55]Eikmeier und Rehm, 1984 (Schimmelpilzmycel/Alginat), [56]Bihari et al., 1984 (Schimmelpilzkonidien/Polyacrylamid), [57]Vogel und Brodelius, 1984 (Pflanzenzellen/Alginat, Agarose), [58]Nakajima et al., 1985 (Pflanzenzellen/Agar, Alginat, Carrageenan) beschrieben, durchgeführt werden.

Daneben können die Mikroorganismen durch Auswahl geeigneter Reaktorparameter zu einer Selbstimmobilisation unter Ausbildung von dichtgepackten Schlammflocken oder -partikeln (granular sludge) veranlasst werden. Dabei ist die Herstellung und Aufrechterhaltung geeigneter Flockungsbedingungen im Reaktor von besonderer Bedeutung, i.e. die Gegenwart von $Ca^{2+}$-Ionen, angepasste Durchmischung sowie die Abwesenheit von hochkonzentriertem nur wenig ausflockendem suspendiertem Material.

Diese Verfahren sind bei Lettinga G et al., 1980 beschrieben.

Bei der Membranabtrennung handelt es sich um die Schaffung definierter, abgegrenzter Räume, in denen die Reaktion(en) abläuft (ablaufen).

Man unterscheidet die folgenden grundsätzlichen Varianten der Membranabtrennung

    a) Mikroenkapsulierung

    b) Liposomentechnik

    c) Anwendung von Biokatalysatoren in Membranreaktoren.

Die vorstehend beschriebenen Immobilisierungsmethoden können auch untereinander kombiniert verwendet werden, wie beispielsweise die Adsorption und die Quervernetzung.

Bevorzugt im Rahmen der vorliegenden Erfindung ist die Immobilisierung der SRM durch adsorptive, ionische oder kovalente Bindung an wasserunlösliche Trägermaterialien, wie z.B. Kohle, Sand, Glas, Plastik, Lavagestein oder Keramik, ohne aber durch diese beispielhafte Aufzählung den Gegenstand dieser Erfindung zu limitieren.

Ganz besonders bevorzugt sind poröse Sintergläser mit einem Durchmesser von 1 mm bis 10 mm [ [29]Aivasidis A. und Wandrey C., 1984], die nach einem speziellen Sinterprozess hergestellt werden und sich durch ein grosses Porenvolumen sowie gleichmässig verteilte Poren auszeichnen, die einen Durchmesser von 0.01 mm bis 1 mm aufweisen. Die Mikroorganismen werden durch diese Sintergläser gleichsam wie durch einen Schwamm aufgesaugt, wobei sie unter Ausbildung von Mikrokolonien innerhalb des Glaskörpers weiterwachsen können.

Ebenfalls bevorzugt ist die Verwendung von porösem Lavagestein mit einem Durchmesser von 0.5 cm bis 5 cm, insbesondere von 2 cm bis 3 cm, und einem Porendurchmesser von 0.1 mm bis 10 mm.

Die Inkubation der im Rahmen dieser Erfindung verwendeten SRM mit Abfallschwefelsäure kann mit Hilfe von Verfahren durchgeführt werden, die in der Abwassertechnologie üblich und seit langem etabliert sind.

Dabei können sowohl vollständig durchmischte als auch nur teilweise oder nicht-vollständig durchmischte Systeme verwendet werden.

In erster Linie sind hier verschiedene Reaktortypen zu nennen, wie beispielsweise Mischreaktoren mit Membranabscheidern, Mischreaktoren mit Lamellenabscheidern, Wirbelschicht-Reaktoren, UASB-Reaktoren (Upflow Anaerobic Sludge Blanket Reaktor), Festbett-Reaktoren, sowie insbesondere Festbett-Umlaufreaktoren, ohne jedoch darauf beschränkt zu sein.

Verschiedene dieser Reaktortypen sind bereits im grosstechnischen Masstab realisiert worden [ [59]Heijnen J J, et al., EWPCA Conference on Anaerobic Waste Water Treatment, 15-19 Sept. 1986, Amsterdam, pp. 159-173, 1986].

Der im Rahmen der vorliegenden Erfindung bevorzugte Reaktortyp ist der Festbett-Umlaufreaktor.

In einem Festbett-Umlaufreaktor liegt die Biomasse in immobilisierter Form, gebunden an kleine Partikel, vor. Diese mit Biomasse umschlossenen Partikel werden dabei durch einen aufwärts gerichteten, ständig zirkulierenden Wasserstrom angeströmt, sodass das Trägermaterial ständig in einem fluidisierten Zustand gehalten wird. Dabei wird kontinuierlich neues Sulfat-haltiges Medium zudosiert.

Dieses Reaktor-System besitzt gegenüber herkömmlichen Verfahren zahlreiche Vorteile, wie z.B. eine höhere Reinigungsleistung aufgrund der hohen Biomassekonzentration und -aktivität, geringer Raumbedarf aufgrund seiner kompakten Bauweise, u.a.m. [siehe [29]Aivasidis and Wandrey, 1984].

Um einen Hochleistungsreaktor für die Sulfat-Reduktion zu betreiben, ist es notwendig, den gebildeten Schwefelwasserstoff kontinuierlich aus dem System zu entfernen.

Der anaerobe Abbau organischer Verbindungen durch Mikroorganismen endet in der Bildung von $CO_2$ und $CH_4$ (Disproportionierungs-Reaktion der organischen Moleküle in $CO_2$, der maximal oxidierten und $CH_4$, der maximal reduzierten Kohlenstoff-Verbindungen). Wenn Sulfate oder Nitrate im System vorhanden sind, entstehen anstelle von $CH_4$ $H_2S$ bzw. $NH_3$, welche für methanogene und acetogene Mikroorganismen toxisch sind und die optimale Disproportionierung der organischen Verbindungen konkurrenzieren und damit hemmen.

Bereits bei einer freien $H_2S$-Konzentration über 50 ppm sind die Methan-und SR-Bakterien in ihrem Stoffwechsel gehemmt. Indem man den pH des Reaktors gegen 8 hält, kann die totale Sulfid-Konzentration ($HS^-$ + $H_2S$) wegen des $pK_{H_2S/SH^-}$ = 7, maximal die 10fache Menge betragen, also etwa 500 ppm. Realistische Sulfid-Konzentrationen ($H_2S$ + $HS^-$), die noch ein sicheres Betreiben eines Hochleistungsreaktors erlauben, dürften bei 300 ppm liegen, sofern der mittlere pH des Systems zwischen pH-Werten von 7,6 bis 8 gehalten wird.

Bei Verwendung nicht-durchmischter Systeme kann es zur Ausbildung eines natürlichen pH-Gradienten über dem Festbett kommen, der sich auf Werte zwischen pH 7 und pH 9 erstrecken kann.

Zur kontinuierlichen Entfernung des $H_2S$ aus dem System wird das Biogas oder ein Teilstrom davon gewöhnlich aus der Fermentation durch ein Gas-Waschsystem geführt [ [60] Särner E, et al., Aquatech 1986; [61]) Michaelson T und Kloss R, 1987]. Das gereinigte Inert-Gas führt man anschliessend wieder in die Biologie zurück, um damit weiteres $H_2S$ bzw. $NH_3$ aus dem System zu entfernen und so eine ungehemmte Fermentation zu ermöglichen ("Stripping").

Diese auf dem sogenannten "Stripping" von $H_2S$ basierenden Verfahren haben jedoch entscheidende Nachteile, die in erster Linie die Kostenfrage und damit die Rentabilität dieser Verfahren betreffen.

7

Für das Austreiben des $H_2S$ aus dem biologischen System verwendet man in der Regel von Sauerstoff befreite Inert-Gase ($N_2$-Strom), die sehr teuer sind und nur bedingt durch billigere Strippgase ersetzt werden können.

Ein weiterer Gesichtspunkt betrifft die Weiterverarbeitung des ausgetriebenen $H_2S$. Das aus dem Bioreaktor entnommene Gasgemisch enthält nur wenige Prozent $H_2S$, in der Regel zwischen ca. 2 bis 8 Vol %.

Für die weitere Verwertung dieses niedrig konzentrierten $H_2S$ kommen in erster Linie adsorptive Verfahren oder Nassverfahren in Betracht. Bei den oxidativen Nassentschwefelungsverfahren, wie dem relativ aufwendigen Stretford-Verfahren oder dem Giammarco-Vetrocoke-Verfahren wird aus Schwefelwasserstoff wegen der Prozess-Stabilität im ganzen Schwefel-Rezyklierungs-Prozess direkt Elementarschwefel produziert, der jedoch von schlechter Qualität ($< 99,8$ %) und daher nur bedingt für die Schwefelsäureherstellung geeignet ist. $

Qualitätiv besseren Schwefel liefert der Claus-Prozess, der jedoch nur mit hochprozentigen $H_2S$-Strömen arbeitet und damit an eine weitere kostenverursachende Aufkonzentrierung des $H_2S$ geknüpft ist.

Diese Nachteile konnten jetzt im Rahmen dieser Erfindung überraschenderweise durch die Anwendung einfacher Verfahrensmassnahmen überwunden werden.

Die erfindungsgemässen Verfahrensmassnahmen zur Beseitigung des toxischen $H_2S$ aus dem Bio-Reaktor umfassen im wesentlichen die Ausfällung des $H_2S$ als Metallsulfid, sowie das Entfernen des gefällten MeS aus dem Reaktor.

Das auf die Weise gewonnene Metallsulfid kann als Suspension gelagert und/oder direkt zu hochkonzentriertem $H_2S$ weiterverarbeitet werden.

Zur Ausfällung von toxischem $H_2S$ können erfindungsgemäss all diejenigen Schwermetallsalze verwendet werden, die im sauren pH-Bereich löslich sind, im neutralen pH-Bereich aber zu einer Ausfällung in Form des entsprechenden Metallsulfids führen.

Besonders geeignet für die Anwendung in dem erfindungsgemässen Verfahren sind beispielsweise Eisen-, Kobalt-, Nickel-, Mangan- oder Zink-Salze, wobei diese beispielhafte Aufzählung aber keine Limitierung des Erfindungsgegenstandes darstellt.

Ganz besonders bevorzugt im Rahmen dieser Erfindung ist die Verwendung von Eisensalzen.

Hierbei ist zu beachten, dass der pH-Wert im Verlauf der Metallsulfat-Fällung vorübergehend leicht absinken kann, was aber z.B. durch eine partielle $CO_2$-Entfernung sehr einfach wieder ausgeglichen werden kann.

Die Fällung des im Bioreaktor vorliegenden $H_2S$ erfolgt durch dosierte Zugabe einer $MeSO_4$-Lösung, insbesondere durch Zugabe einer $FeSO_4$-Lösung, zum Sulfid-haltigen Ausfluss der biologischen Reaktionseinheit.

Die Zudosierung der $MeSO_4$-Lösung muss genau erfolgen und kann beispielsweise mit einem der folgenden Systeme überwacht werden:

a) Mit einer $H_2S$ sensitiven Elektrode kann $MeSO_4$ zugegeben werden, bis alles oder fast alles $H_2S$ (z.B. bis 10 ppm) zu MeS reagiert hat.

b) Mit einer on-line-Sulfat-Bestimmungsanlage kann soviel Sulfat als $MeSO_4$ zugegeben werden, dass immer gleichviel $SO_4$ im System ist. Fehlendes Sulfat ist als Sulfid vorhanden und wird von Me-Ionen gebunden.

c) Ueber die Potentialbestimmung (Leitfähigkeitsmessung):
Dem Abfluss der Biologie wird soviel Me-Sulfat-Lösung zugegeben, bis das Potential zunimmt (bedingt durch den Potentialsprung MeS $\leftrightarrow$ $MeSO_4$).

d) Ueber die Messung der freien $Me^{2+}$-Ionen-Konzentration, die ansteigt, sobald alles Sulfid als MeS gefällt ist.

Die Fällung des Metallsulfids kann durch Zugabe eines Flockungsmittels, wie es üblicherweise in der Abwassertechnik zur Fällung von Biomasse, Chemikalien etc. eingesetzt wird, verbessert werden.

Besonders geeignet im Rahmen dieser Erfindung sind organische Flockungsmittel, insbesondere solche auf Polyacrylamidbasis, wobei diese Flockungsmittel erfindungsgemäss sowohl kationischen als auch anionischen Charakter haben können.

Kationische Flockungsmittel auf Polyacrylamidbasis können beispielsweise durch Quervernetzung der Acrylamid-Polymere mit negativ geladenen $SO_3^{2-}$ Gruppen entstehen, während bei den entsprechenden anionischen Flockungsmitteln die Quervernetzung z.B. über positiv geladene Stickstoffatome vermittelt werden kann.

Diese zuvor näher charakterisierten Flockungsmittel sind dem Fachmann auf dem Gebiet der Abwassertechnologie bekannt und können z.B. unter der Markenbezeichnung Superfloc A 150®, Praestol 2935®, Separan $AP_{30}$® (anionisch) oder Praestol 444k® (kationisch) kommerziell bezogen werden.

Selbstverständlich kann auch jedes andere, für die Anwendung in dem erfindungsgemässen Verfahren geeignete Flockungsmittel verwendet werden.

Die Abtrennung des gefällten Metallsulfids, sowie des eventuell vorhandenen Bioschlamms aus der biologischen Reaktionseinheit erfolgt unter Verwendung bekannter Trennverfahren, wie z.B. durch Sedimentation, Flotation, Filtration und/oder Zentrifugation.

Besonders bevorzugt im Rahmen dieser Erfindung ist die Sedimentation des gefällten Metallsulfids, die gegebenenfalls durch eine Filtration, insbesondere durch eine Ultrafiltration zur Feinreinigung des Ueberstandes und eine Dekantierzentrifugation zur Entwässerung des abgesetzten Eisensulfids ergänzt werden kann.

Das im Verlauf dieses Trennprozesses anfallende $H_2S$-arme, sulfathaltige Permeat wird anschliessend gegebenenfalls von überschüssigem $CO_2$ befreit, mit einer geeigneten organischen C-Quelle sowie Nährsalzen versetzt und in den Bioreaktor zurückgeführt.

Der Metallsulfid-Schlamm, welcher den reduzierten Schwefel enthält, kann entweder unter Inertgas zwischengelagert oder in einer dritten, rein chemischen Verfahrensstufe, direkt zu hochwertigen, anorganischen Produkten umgewandelt werden.

Dabei wird der abgetrennte Metallsulfid-Schlamm zunächst mit Abfallschwefelsäure versetzt, wobei ein hochprozentiges Sauergas entsteht, welches einen $H_2S$ Anteil von 80 bis 100 Vol % enthält.

Dieser hochkonzentrierte Schwefelwasserstoff kann anschliessend mit Hilfe von Standardverfahren in hochwertige, anorganische Produkte umgewandelt werden. Beispielhaft seien hier die Weiterverarbeitung zur Elementarschwefel nach dem Claus-Prozess, die Verbrennung zu Schwefeldioxid ($SO_2$), das anschliessend zu frischer, gebrauchsfertiger Schwefelsäure aufgearbeitet werden kann sowie das Verfahren von Sconce und Berlinghoff zur Herstellung von $Na_2S$ genannt, ohne dadurch den Erfindungsgegenstand zu limitieren.

Im "Claus-Prozess" wird das hochkonzentrierte $H_2S$ zu Elementarschwefel gemäss der folgenden Grundreaktion verbrannt:

$$2H_2S + O_2 \rightarrow S_2 + 2H_2O \quad \Delta H = 315,1 \text{ kJ}$$

Die Reaktion ist stark exotherm und damit sehr temperaturabhängig. Der im "Claus-Prozess" anfallende elementare Schwefel kann dann gemäss allgemein bekannten Standardverfahren zu Schwefelsäure weiterverarbeitet werden.

Das bereits 1941 von Sconce und Berlinghoff vorgeschlagene Verfahren zur Herstellung von hochprozentigen Natriumsulfid- oder -hydrogensulfid-Lösungen aus $H_2S$-Reichgas besteht im wesentlichen aus der Umsetzung einer wässrigen Natronlauge mit Schwefelwasserstoff nach Gleichung 1) und mit einem weiteren Aequivalent $H_2S$ nach Gleichung 2).

1) $2NaOH \cdot 0,95 H_2O + H_2S \rightarrow Na_2S \cdot 3,9H_2O$
2) $Na_2S \cdot 3,9H_2O + H_2S \rightarrow 2NaSH \cdot 1,95H_2O$

Natriumsulfid- und Natriumhydrogensulfidlösungen finden vor allem in der Farbenchemie ihre Anwendung, wo sie zur schonenden, partiellen Reduktion von Nitro- und Nitroso-Verbindungen eingesetzt werden.

Das erfindungsgemässe Verfahren erlaubt neben der Entsorgung normal belasteter Abfallschwefelsäure auch die Verwendung kritischer Schwermetall-haltiger Schwefelsäuren zur Aufarbeitung des Metallsulfids.

Falls die Abfallschwefelsäuren, welche zur Aufarbeitung des Metallsulfids verwendet werden, mit Schwermetallen wie Kupfer, Quecksilber, Blei, Cadmium, Wismut, Arsen, Antimon, Zinn etc. verunreinigt sind, deren Sulfide im sauren Milieu nicht löslich sind, so reichern sich diese als Schwermetall-Sulfide an. Es ist also möglich, auch Schwermetall-haltige Schwefelsäuren zu entsorgen. Dabei wird die Biologie von den Schwermetallen bzw. von Schwermetall-Sulfiden nicht beeinflusst, da diese bereits bei der Aufarbeitung des Metallsulfid-Schlammes immobilisiert und ausgefällt werden. Die konzentrierten, unlöslichen Schwermetall-Sulfid-Lösungen können mit Hilfe alternativer anderweitiger Methoden aufgearbeitet und rezykliert oder deponiert werden.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn, es würde speziell darauf hingewiesen.

Nichtlimitierende Ausführungsbeispiele

Beispiel 1:

A) Nährmedium

Für die Anreicherung sulfatreduzierender Bakterien wird ein Medium mit der folgenden Zusammensetzung verwendet:

| | |
|---|---|
| $Na_2SO_4$ | 2 g/$\ell$ |
| $KH_2PO_4$ | 0,2 g/$\ell$ |
| $NH_4Cl$ | 0,3 g/$\ell$ |
| $MgCl_2 \cdot 6H_2O$ | 0,2 g/$\ell$ |
| $CaCl_2 \cdot 2H_2O$ | 0,2 g/$\ell$ |
| Spurenelement-Lösung | 1 m$\ell$/$\ell$ |
| Vitamin-Lösung | 5 m$\ell$/$\ell$ |
| pH = 7,4 | |
| Na-Acetat | 0,5 g/$\ell$ |
| Resazurin (0,2%) | 1 m$\ell$/$\ell$ |

Die Spurenelemente-Lösung setzt sich aus folgenden Komponenten zusammen:

| | |
|---|---|
| $H_2SO_4$ (50 %$^v$/v) | 2 m$\ell$/$\ell$ |
| $FeSO_4 \cdot 7H_2O$ | 1,5 g/$\ell$ |
| $H_3BO_3$ | 60 mg/$\ell$ |
| $MnSO_4 \cdot H_2O$ | 100 mg/$\ell$ |
| $CoCl_2 \cdot 6H_2O$ | 120 mg/$\ell$ |
| $NiCl_2 \cdot 6H_2O$ | 25 mg/$\ell$ |
| $ZnSO_4 \cdot 7H_2O$ | 100 mg/$\ell$ |
| $(NH_4)_6Mo_7O_{24} \cdot 4H_2O$ | 50 mg/$\ell$ |
| $CuSO_4 \cdot 5H_2O$ | 20 mg/$\ell$ |

Die Vitamin-Lösung setzt sich aus folgenden Komponenten zusammen:

| | |
|---|---|
| Biotin | 2 mg/$\ell$ |
| Pyridoxamin | 50 mg/$\ell$ |
| Thiamin | 10 mg/$\ell$ |
| Nicotinsäure | 20 mg/$\ell$ |
| Panthotensäure | 5 mg/$\ell$ |
| Cyanocobalmin | 20 mg/$\ell$ |
| p-Aminobenzoesäure | 10 mg/$\ell$ |

Dieses sogenannte Startermedium enthält eine 0,2 %ige Resazurin-Lösung als Indikatorfarbstoff, der das Vorliegen anaerober Verhältnisse durch einen Farbumschlag anzeigt.

B) Reaktorsystem

Als Reaktorsystem für die Sulfat-Reduktion wird ein Festbett-Umlauf-Bioreaktor verwendet, der luftdicht abgeschlossen werden kann und ein Schüttvolumen von ca. 1 $\ell$ aufweist. Verschiedene axial angeordnete Stutzen erlauben das Anbringen von Sonden zur Messung und Regelung von Temperatur und pH-Wert sowie von Einrichtungen zur Probe- und Produktentnahme und der Rezirkulation des Reaktormediums.

Die Lineargeschwindigkeit des Zulaufstromes liegt bei ca. 2 bis 40 m/h, was ausreicht, um eine partielle Fluidisierung der Schüttung zu erreichen.

Die Beladung des Reaktors erfolgt mit Kugeln aus porösem Sinterglas sowie einem unter Punkt A bezeichneten Startermedium.

C) Immobilierung der SRB

Die Immobilierung der SRB erfolgt mit Hilfe von Kugeln aus porösem Sinterglas der Firma Schott Werke AG, Mainz, BRD, die einen Durchmesser von 3 mm aufweisen.

Die Sulfat-reduzierenden Bakterien werden von dem porösen Trägermaterial wie von einem Schwamm aufgesaugt und besiedeln dessen innere Hohlräume unter Ausbildung von neuen Mikrokolonien.

Die besondere Struktur dieser Sinterglaspartikel, insbesondere deren durchgängiges Hohlraumsystem gestattet einen limitierungsfreien Stofftransport bei guter Durchströmung. Auch Produkte der Zellyse, die in der Regel toxisch sind für die noch aktiven Zellen und diese schädigen können, werden leicht ausgetragen und beeinflussen das Wachstum und die Stoffwechselaktivität der SRB daher nicht.

Der Auslauf des Reaktorsystems wird anaerob in ein luftdicht verschlossenes Gefäss geleitet, wo unter pH-kontrollierten Bedingungen Eisensulfat-Lösung zur Ausfällung von Sulfid zugegeben wird (siehe Punkt F). Nach der Sedimentation des Eisensulfids wird der grösste Teil der Lösung unter strikt anaeroben Bedingungen wieder in den Bioreaktor zurückgeführt, während ein kleiner Teil verworfen wird.

D) Anfahrprozess; Anreicherung der SRB

Der Bioreaktor wird mit dem Startermedium gefüllt, auf 35° C erwärmt und mit 1 ml Klärschlamm beimpft. Die Flüssigkeit im Reaktor wird 24 Stunden zirkuliert. In dieser Zeit wird das im Startermedium enthaltene Acetat von fakultativ anaerobem und aeroben Mikroorganismen unter Sauerstoffzehrung oxidiert. Der Redoxindikator färbt sich von blau zu rot und farblos. Nach 24 Stunden wird der anaerobe, farblose Bioreaktor mit 10 ml anaerobem Faulschlamm beimpft. Als Impfgut dient Sulfid-produzierender Klärschlamm aus der Kläranlage ARA Füllinsdorf in Füllinsdorf, Schweiz.

Neben Anreicherungskulturen Sulfat-reduzierender Bakterien aus Faulschlämmen können in dem erfindungsgemässen Verfahren auch die folgenden Bakterien Stämme, einzeln oder in Kombination, eingesetzt werden.

Desulfobacter postgatei DSM 2034, Desulfobacter postgatei DSM 2553, Desulfotomaculum acetoxidans DSM 771, die bei der Deutschen Sammlung von Mikroorganismen (DSM) in Göttingen, BRD, bezogen werden können.

E) Kontinuierliche Phase

Nach einer Anlaufphase von drei Tagen wird der Bioreaktor kontinuierlich mit frischem Medium beschickt, das sich in seiner Zusammensetzung von dem unter Punkt A angegebenen Startermedium in folgenden Punkten unterscheidet:

| $Na_2SO_4$ | — |
| Abfallschwefelsäure ($SO_4$) | 4,8 g/l - 16,5 g/l (siehe Tab. 1) |
| Essigsäure | 1,26 g/l - 5,4 g/l (siehe Tab. 1) |
| Vitaminlösung | 2 ml (wird separat alle 2-3 Tage zugegeben) |

pH < 2

Die dem Medium zugegebenen Acetat- und Sulfatmengen ändern sich ständig mit steigender Umsatzleistung der Sulfat-reduzierenden Mikroorganismen (siehe Tabelle 1). Die durchschnittliche Sulfatkonzentration im volldurchmischten Bioreaktor bleibt aber konstant und liegt bei ca. 10 g/l. liegt bei ca. 10 g/l.

Der Bioreaktor wird nur sehr langsam und kontinuierlich mit dem Abfallschwefelsäuremedium gefüttert (Anfangsdosis 5-50 ml/Tg).

Bei steigender Sulfidproduktion und abnehmender Sulfatkonzentration im Bioreaktor wird der Zulauf langsam erhöht.

Der mittlere pH-Wert im Bioreaktor bleibt während der gesamten Versuchsdauer stabil bei einem Wert von pH 7.6.

Die $H_2S$-Konzentration steigt bis auf Werte von 150 mg/$\ell$ an, was bei einem pH 7.6 einer freien $H_2S$-Konzentration von ca. 40 mg/$\ell$ entspricht.

F) Ausfällung von $H_2S$

Wenn der Sulfidgehalt im Bioreaktor 20 ppm übersteigt, wird das gebildete $H_2S$ kontinuierlich durch Schwermetallfällung entfernt.

Dabei wird bei konstantem pH-Wert (7.4 bis 7.6) Eisensulfat je nach Bedarf über eine $H_2S$-Mess- und Regelstation zudosiert, sodass die totale Sulfidkonzentration unter 30 mg/$\ell$ sinkt. Dem pH-statisch gerührten Mischkessel wird zudem das kathionische Flockungsmittel Praestol 444K® beigegeben (10 mg/$\ell$). Dadurch kann die Sedimentation von FeS beschleunigt werden. Die Suspension wird anschliessend in ein Sedimentationsbecken geleitet, wo sich das Eisensulfid absetzt. Der schwachtrübe Ueberstand mit einem Trockengehalt von 75 mg/$\ell$ wird wieder dem Bioreaktor zugeführt.

Der aus dem Sedimentationsgefäss abgezogene flockige Niederschlag hat einen Feststoffgehalt von 65 g/$\ell$. Der Feststoff lässt sich gut filtrieren, wobei ein klares Filtrat erhalten wird. Der Filterkuchen weist ein Trockengehalt von ca. 100 g/$\ell$ auf, wobei es sich um eine thixotrophe, stichfeste Paste handelt. Diese Eisensulfidpaste wird anschliessend unter Inert-Gas aufbewahrt, da sonst eine Oxidation des Schwefels mit Luftsauerstoff erfolgt und gelbes Eisenhydroxid gebildet wird.

Die Umsatzleistungen sowohl für Essigsäure als auch für das eingesetzte Sulfat liegen bei über 80 %. Bei der Verwendung von Methanol als organische C-Quelle entsteht im wesentlichen $CH_4$ als Reduktionsprodukt.

Die maximale spezifische Sulfat-Reduktionsrate beträgt 60 g $SO_{4red}$/$\ell$ Bioreaktor/d. Dies entspricht mindestens der doppelten bisher in der Literatur beschriebenen maximalen Umsatzrate.

Tabelle 1

Acetat als C-Quelle

| Tag | $Vol_{Ein}$ | $Acetat_{Ein}$ | $Acetat_{Aus}$ (als DOC) | $SO_{4Ein}$ | $SO_{4Aus}$ | $SO_4$ abgebaut | Sulfid-Produk-tion | Totale Sulfid Konzentration in Biologie | Spezif. Sulfat-Reduk-tionsrate |
|---|---|---|---|---|---|---|---|---|---|
| | $\ell$/d | g/$\ell$ | g/$\ell$ | g/$\ell$ | g/$\ell$ | % | g/d | mg/$\ell$ | g/$\ell$ Bioreaktor/d |
| 8 | 0,3 | 1,26 | 0,06 | 4,8 | 1,7 | 63 | 0,6 | 10 | 0,9 |
| 26 | 2,0 | 1,53 | 0,26 | 4,6 | 0,3 | 93 | 3,3 | 35 | 9,0 |
| 33 | 2,8 | 2,50 | 0,93 | 7,5 | 1,9 | 75 | 5,6 | 56 | 17,0 |
| 40 | 2,8 | 2,96 | 0,78 | 9,4 | 1,3 | 86 | 8,3 | 50 | 24,5 |
| 47 | 2,8 | 4,30 | 1,18 | 12,8 | 1,6 | 87 | n.g. | 135 | 33,7 |
| 52 | 2,8 | 5,40 | 1,43 | 16,7 | 2,2 | 86 | 15,4 | 190 | 43,4 |
| 54 | 2,8 | 5,40 | 1,82 | 16,5 | 3,2 | 81 | 16,8 | 125 | 40,2 |

Tabelle 2

Bilanz der Schwermetall-Fällung

| Edukte: | |
|---|---|
| Behandelte Abwassermenge aus dem Bioreaktor | 19.8 $\ell$ |
| Mittlere Sulfidkonzentration im Abwasser | 150 mg/$\ell$ |
| Sulfidfracht | 2.97 g |
| FeSO$_4$-Verbrauch (IM-Lösung) | 70 ml |
| **Produkte:** | |
| Konzentration Eisensulfid | 0.45 g/$\ell$ |
| Eisensulfidfracht | 8.95 g/$\ell$ |
| Erwartete Eisensulfidfracht bezüglich Eisenzugabe | 6.16 g/$\ell$ |
| Erwartete Eisensulfidfracht bezüglich Sulfidfracht | 8.17 g/$\ell$ |

Beispiel 2

Die spezifischen Verfahrensparameter wie Medienzusammensetzung, Reaktoraufbau, Impfgut, Anreicherungstechnik, Trägermaterial, Schwermetallfällung des H$_2$S u.a. entsprechen den unter Beispiel 1 gemachten Angaben.

Beispiel 2 beschreibt die Möglichkeit einer Verwendung von Methanol als C-Quelle zur Sulfat-Reduktion, wobei ein Mischmedium Acetat-Methanol in einem Konzentrationsverhältnis von 20:1 verwendet wird. Als Sulfat-Quelle wird Abfallschwefelsäure (25 % V/v) eingesetzt, die bei der Farbstoff-Produktion anfällt und Spuren von organischen Verunreinigungen enthält. Als Reaktor wird ein 10 $\ell$ Festbett-Umlaufreaktor verwendet, der mit Kugeln (Ø 3 mm) aus porösem Sinterglas gefüllt wird, auf welchen sich die Mikroorganismen ansiedeln. Der Reaktor wird angefahren, indem das anaerobe System mit ca. 10 m$\ell$ Impfmaterial aus dem 1 $\ell$ Reaktor (Versuch 1) sowie mit 1 $\ell$ einer Kultur von Desulfotomaculum orientis, (DSM 765) einem Methanol-oxidierenden Sulfat-Reduzenten (Wenk et al. 1987), versetzt wird. Nach 3 Tagen beginnt man, den Reaktor kontinuierlich mit neuem Substrat und Abfallschwefelsäure bzw. Sulfat zu beschicken. Der Betriebs-pH beträgt dabei 7,4 bis 7,9.

Die maximale Sulfat-Reduktionsrate liegt bei 15 g SO$_4$/$\ell$Bioreaktor/d. Als Nebenprodukt entstehen grosse Mengen an Methan.

Tabelle 3

Methanol-Acetat als C-Quelle

| Tag | $Vol_{Ein}$ | $Acetat_{Ein}$ | $Methanol_{Ein}$ | $DOC_{Aus}$ | $CH_4$-Produk-tion | $SO_{4Ein}$ | $SO_{4Aus}$ | $SO_4$ abgebaut | Sulfid Produktion | Spezif. Sulfat-Re-duktionsrate |
|---|---|---|---|---|---|---|---|---|---|---|
| | $\ell$/d | g/$\ell$ | mg/$\ell$ | | | g/$\ell$ | g/$\ell$ | % | g/d | g/$\ell$ Bioreaktor/d |
| 10 | 5,6 | 0,2 | 2,0 | 27 | – | 3,4 | 1,9 | 44 | 3,5 | 0,8 |
| 24 | 5,8 | 0,8 | 4,0 | 51 | – | 3,3 | 1,6 | 52 | 5,5 | 1,0 |
| 40 | 37,0 | 0,8 | 4,0 | 309 | + | 3,5 | 1,9 | 46 | 20,8 | 5,7 |
| 46 | 38,0 | 0,8 | 4,0 | 447 | + | 3,5 | 1,6 | 54 | 23,5 | 7,1 |
| 57 | 40,0 | 0,8 | 4,0 | 160 | + | 3,4 | 1,2 | 65 | 27,7 | 8,5 |
| 70 | 75,0 | 0,8 | 4,0 | 440 | + | 2,9 | 1,1 | 62 | 51,7 | 12,6 |
| 73 | 58,0 | 0,8 | 6,0 | 814 | + | 4,2 | 1,6 | 62 | 70,1 | 15,6 |

EP 0 356 384 A2

Literaturverzeichnis

1) (Burgess S.G. and Wood L.B., J.Sci.Food Agric., 12: 326-335, 1961;

2) Ueki et al., Gen.Appl.Microbiol., 27: 457-464, 1981);

3) (Cork D. and Cusanovich M., Dev.Ind.Microbiol., 20: 591-602, 1979;

4) Cork D., Dev.Ind.Microbiol., 23: 379-387, 1982;

5) Cork D. et al., Appl.Env.Microbiol., 45: 913-918, 1983;

6) Cork D., Mikrobielles Verfahren zur Umwandlung von Gips in elementaren Schwefel, Offenlegungsschrift DE-OS 33 28 500 A1, BRD;

7) Cork D. et al., Appl.Env.Microbiol., 49(2): 269-272, 1985;

8) Cork D., Biocatalytic Production of Sulfur from Process Waste Streams, Biotech. and Bioeng.Sym. No. 16: 149-162, 1986;

9) Grimm D. et al., Dev.Ind.Microbiol., 25: 709-716, 1984;

10) Olthof M. et al., DE-OS 34 22 959 A1, BRD;

11) Maree J.P. and Strydom W.F., Water Res., 19(9): 1101-1106, 1985;

11a) Mulder A., Anaerobic purification of waste water containing sulphate and organic material, EP-A 241,999;

12) Pfennig N et al, (1981) "The Dissimilatory Sulfate-reducing Bacteria", in: "The Prokaryotes" - A Handbook of Habitats, Isolation and Identification of Bacteria", Vol. 1 Springer Verlag, Berlin;

13) Trüper HG (1984) "Microorganisms and the Sulfur Cycle" in: Müller A and Krebs B, "Sulfur, its Significance for Chemistry, for the Geo-, Bio- and Cosmosphere and Technology", Vol. No. 5, Elsevier, Amsterdam;

14) Widdel F (1980), "Anaerober Abbau von Fettsäuren und Benzolsäure durch neu isolierte Arten Sulfat-reduzierender Bakterien, Dissertation, Universität Göttingen, BRD;

15) Pfennig N and Biebl H, Arch.Microbiol., 110: 3-12, 1976;

16) Laanbrock H, et al., Arch.Microbiol., 133: 178-184, 1982;

17) Widdel F and Pfennig N, Arch.Microbiol. 129: 395-400, 1981;

18) Widdel F and Pfennig N, Arch.Microbiol., 131: 360-365, 1982;

19) He S, Biochem.Biophys.Res.Comm., 135(3): 1000-1007, 1986;

20) Badziong W and Thauer R, Arch.Microbiol., 117: 209-214, 1978;

21) Schlegel H G, Allgemeine Mikrobiologie, 6. Auflage, Thieme Verlag, Stuttgart, 1986;

22) Badziong W et al., Arch.Microbiol., 116: 41-49, 1978;

23) Antloga K M, and Griffin W M, Dev.Ind.Microbiol., 26: 597-610, 1985;

24) Widdel F and Pfennig N, Arch.Microbiol., 112: 119-122, 1977;

25) Zeikus J G et al., J.Gen.Microbiol., 129: 1159-1169, 1983;

26) Robinson J A and Tiedje D M, Arch.Microbiol., 137: 26-32, 1984;

27) Hungate R E, "A Roll-Tube Method for Cultivation of strict Anaerobes", in: Norris, Ribbons, Methods in Microbiology, Vol. 3B, 117-132, Academic Press, N.Y., 1969;

28) Braun M and Stolp H, Arch.Microbiol., 142: 77-80, 1985;

29) Aivasidis und Wandrey Berichte der Kernforschungsanlage Jülich, Nr. 1900, Institut für Biotechnologie 2 Jül-1900, 1984;

30) Mc Carty und Smith PL and Smith DP, Environ.Sci.Technol. 20(12): 1200-1206,1986;

31) Baumann G, Korrespondenz Abwasser, 1: 28.37, 1987;

32) van Haecht et al, Biotech.Bioeng., 26: 217-224, 1985;

33) Black et al, Biotech.Bioeng., 26: 134-141, 1984;

34) Wiegel J and Dykstra M, Appl.Microbiol.Biotech., 20: 59-65, 1984;

35) Förberg C and Häggström L, "Adsorbed cell systems controlled by the nutrient dosing technique." In: 3rd Eur.Congr.Biotech. Bd. 2. Verlag Chemie, Weinheim, pp. 115-120, 1984;

36) Ehrhardt HM and Rehm H J, Appl.Microbiol.Biotech., 21: 32-36, 1985;

37) DiLuccio R C and Kirwan D J, Biotech.Bioeng., 26: 87-91, 1984; 38) Giard D J et al., Biotech.Bioeng., 21: 433-442, 1979;

39) Messing R A and Oppermann R A, Biotech.Bioeng., 21: 49-58, 1979;

40) Romanovskaya V A, Karpenko V I, Pantskhava E S, Greenberg T A, Malashenko Y R (1981) Catalytic properties of immobilized cells of methane-oxidizing and methanogenic bacteria. In: Moo-Young M (Hrsg) Advances in Biotechnology, Bd. 3. Pergamon, Toronto, S. 367-372.

41) Navarro J M and Durand G, Eur.J.Appl.Microbiol.Biotech., 4: 243-254, 1977;

42) Chipley J R, Microbiol., 10: 115-120, 1974; 43) Gainer J L et al., Biotech.Boioeng.Symp., 10: 35-42, 1980;

44) Jack T R and Zajic J E Biotech.Bioeng., 19: 631, 1977;

45) Jirku V et al., Biotech.Lett., 2: 509-513, 1980;

46) Shimizu S et al., J.Ferm.Technol., 53: 77-83, 1975;

47) De Rosa M et al., Biotech.Lett., 3: 183-188, 1981;

48) Mazumder T K et al., Appl.Microbiol.Biotech. 21: 154-161, 1985;

49) Bettmann H and Rehm H J, Appl.Microbiol.Biotech., 20: 285-290, 1984;

50) Umemura I et al., Appl.Microbiol.Biotech. 20: 291-295, 1984;

51) Karube I et al., Biotech.Bioeng., 21: 253-260, 1979;

52) Cantarella M et al., Appl.Microbiol.Biotech. 20: 233-237, 1984;

53) Qureshi N and Tamhane D V Appl.Microbiol.Biotech., 21: 280-281, 1985;

54) Deo Y M and Gaucher G M Biotech.Bioeng., 26: 285-295, 1984;

55) Eikmeier H and Rehm H J Appl.Microbiol.Biotech., 20: 365-370, 1984;

56) Bihari V et al., Biotech.Bioeng. 26: 1403-1408, 1984;

57) Vogel H J and Brodelius P, J.Biotech., 1: 159-170, 1984;

58) Nakajima H et al., J.Biotech. 2: 107-111, 1985;

59) Heijnen J J, et al., EWPCA Conference on Anaerobic Waste Water Treatment, 15-19 Sept. 1986, Amsterdam, pp. 159-173, 1986;

60) Särner E, et al., "New Technique for Control of $H_2S$ Toxicity and Recovery of Sulphur in Anaerobic Treatment, SPCI 87 Conference, Stockholm, April 7-10, 1987;

61) Michaelson T and Kloss R, GWF.Wasser.Abwasser, 128(4): 242-250, 1987.

## Patentansprüche

1. Verfahren für das Recycling von Abfallschwefelsäure, dadurch gekennzeichnet, dass man Abfallschwefelsäure ohne vorhergehende Neutralisation

a) in einer ersten, rein biologischen Verfahrensstufe mit Hilfe Sulfat-reduzierender Mikroorganismen unter strikt anaeroben Bedingungen zu Sulfid reduziert,

b) das für die Sulfat-reduzierenden Mikroorganismen toxische Sulfid durch Zugabe von Metall-Salzen aus dem Inkubationsmedium entfernt und

c) das gefällte Metallsulfid in einer dritten Verfahrensstufe mit ausschliesslich chemisch/physikalischen Methoden zu wiederverwertbaren Rohmaterialien aufarbeitet.

2. Verfahren gemäss Anspruch 1, dadurch gekennzeichnet, dass es sich bei besagten Rohmaterialien um Schwefelsäure handelt.

3. Verfahren gemäss Anspruch 1, das durch die folgenden Verfahrensmassnahmen gekennzeichnet ist:

1) Reduktion von Sulfat aus Abfallschwefelsäure durch Sulfat-reduzierende Mikroorganismen zu $H_2S$;

2) Kontinuierliches Entfernen von toxischem $H_2S$ aus dem Bio-Reaktor-Medium durch dosierte Zugabe von Metallsulfat, das mit dem anfallenden Sulfid zu Metallsulfid (MeS) reagiert.

3) Abtrennen des ausgefällten Metallsulfids mit Hilfe an sich bekannter Trennverfahren.

4) Versetzen der entfernten MeS-Suspension mit Abfallschwefelsäure unter Freisetzung hochkonzentrierten $H_2S$-Gases.

5) Direkte Aufoxidation von $H_2S$ zu $H_2SO_4$, und Zurückführen der in 4) entstehenden $MeSO_4$-Lösung in den Prozesskreislauf zum Ausfällen von Sulfid aus dem Bioreaktor-Medium und zur weiteren Zugabe von Sulfat als Substrat.

6) Versetzen der aus 2 und 3 resultierenden $H_2S$-armen, Sulfat-haltigen und gegebenenfalls von $CO_2$ befreiten Lösung mit einer C-Quelle sowie geeigneten Nährsalzen und Zurückführen in Verfahrensstufe 1.

4. Verfahren gemäss Anspruch 1, dadurch gekennzeichnet, dass es sich bei den Sulfat-reduzierenden Mikroorganismen um Sulfat-reduzierende Bakterien handelt.

5. Verfahren gemäss Anspruch 4, dadurch gekennzeichnet, dass es sich bei den Sulfat-reduzierenden Bakterien um Vertreter aus den Gattungen Desulfovibrio, Desulfotomaculum, Desulfobacter, Desulfomonas, Desulfobulbus, Desulfococcus, Desulfonema, Desulfosarcina, oder Thermosulfobacterium handelt.

6. Verfahren gemäss Anspruch 4, dadurch gekennzeichnet, dass es sich bei besagten Sulfat-reduzierenden Bakterien um eine Bakterien-Mischkultur handelt.

7. Verfahren gemäss Anspruch 6, dadurch gekennzeichnet, dass es sich bei besagter Bakterien-Mischkultur um eine Anreicherungs-Kultur Sulfat-reduzierender Bakterien von anaeroben Standorten handelt, die gegebenenfalls einen Sulfat-Ueberschuss sowie ein genügend grosses Angebot an Energiequellen aufweisen.

8. Verfahren gemäss Anspruch 7, dadurch gekennzeichnet, dass es sich bei besagter Mischkultur um eine Anreicherungskultur aus Sulfat-haltigen Klarschlämmen handelt.

9. Verfahren gemäss Anspruch 7, dadurch gekennzeichnet, dass man zur Anreicherung Sulfat-reduzierender Bakterien

a) zunächst ein Sulfat-haltiges Startermedium, das neben einer geeigneten Kohlenstoff- und Energiequelle, die für die Vermehrung und Erhaltung der Mikroorganismen essentiellen mineralischen Stoffe bzw. Elemente in ihren Verbindungen sowie verschiedene essentielle Vitamine u.a. Zusatzstoffe enthält, mit Material beimpft, das neben aeroben und fakultativ anaeroben Mikroorganismen gegebenenfalls auch Sulfat-reduzierende Bakterien enthält

b) das Impfgut in besagtem Startermedium solange inkubiert, bis strikt anaerobe Verhältnisse vorliegen

c) das anaerobe System unter strikt anaeroben Bedingungen mit anaerobem Material beimpft, welches Sulfat-reduzierende Bakterien enthält und

d) nach einer Adaptationsphase von 2-10 Tagen kontinuierlich frisches Medium mit Sulfat aus Abfallschwefelsäure zugibt.

10. Verfahren gemäss Anspruch 1, dadurch gekennzeichnet, dass besagte Sulfat-reduzierende Mikroorganismen in immobilisierter Form vorliegen.

11. Verfahren gemäss Anspruch 10, dadurch gekennzeichnet, dass besagte Mikroorganismen an ein festes Trägermaterial ausgewählt aus der Reihe Kohle, Sand, poröses Sinterglas, Plastik, Lavagestein oder Keramik immobilisiert sind.

12. Verfahren gemäss Anspruch 10, dadurch gekennzeichnet, dass die Immobilisierung der Sulfat-reduzierenden Mikroorganismen in Form dichtgepackter Schlammpartikel ('granular sludge') erfolgt.

13. Verfahren gemäss Anspruch 1, dadurch gekennzeichnet, dass man ein nicht vollständig durchmischtes Reaktorsystem verwendet.

14. Verfahren gemäss Anspruch 1, dadurch gekennzeichnet, dass die biologische Sulfat-Reduktion in einem Bio-Reaktor ausgewählt aus der Gruppe der Mischreaktoren mit Membranabscheidern, Mischreaktoren mit Lamellenabscheidern, Wirbelschicht-Reaktoren, UASB-Reaktoren (Upflow Anaerobic Sludge Blanket Reaktor) Festbett-Reaktoren, sowie Festbett-Umlaufreaktoren durchgeführt wird.

15. Verfahren gemäss Anspruch 1, dadurch gekennzeichnet, dass man die Inkubation der Abfallschwefelsäure mit den Sulfat-reduzierenden Mikroorganismen in einem Medium vornimmt, das neben einer geeigneten Kohlenstoff- und Energiequelle, die für die Vermehrung und Erhaltung der Mikroorganismen essentiellen mineralischen Stoffe bzw. Elemente in ihren Verbindungen sowie verschiedene essentielle Vitamine u.a. Zusatzstoffe enthält.

16. Verfahren gemäss Anspruch 15, dadurch gekennzeichnet, dass sich die Salzkonzentration im Medium bei einem Wert zwischen 2 g/$\ell$ und 10 g/$\ell$ hält.

17. Verfahren gemäss Anspruch 15, dadurch gekennzeichnet, dass man als Kohlenstoff- und Energiequelle Lactat, Acetat oder Propionat verwendet.

18. Verfahren gemäss Anspruch 17, dadurch gekennzeichnet, dass man bei Einsatz von Carbonsäuren das entstehende $CO_2$ aus dem Kulturmedium entfernt.

19. Verfahren gemäss Anspruch 15, dadurch gekennzeichnet, dass man als Kohlenstoff- und Energiequelle Methanol oder Acetat oder ein Gemisch aus Methanol und Acetat verwendet.

20. Verfahren gemäss Anspruch 15, dadurch gekennzeichnet, dass die Konzentration der Kohlenstoff-, und Energiequelle zwischen 0.1 g/$\ell$ und 25 g/$\ell$ liegt.

21. Verfahren gemäss Anspruch 15, dadurch gekennzeichnet, dass der mittlere pH-Wert des Kultivierungsmediums zwischen pH 7 und pH 9 liegt.

22. Verfahren gemäss Anspruch 1, dadurch gekennzeichnet, dass die Prozess-Temperatur für die Sulfat-Reduktion zwischen 20° C und 50° C beträgt.

23. Verfahren gemäss Anspruch 3, dadurch gekennzeichnet, dass die dosierte Zugabe von Metall-Sulfat durch eines der folgenden Systeme überwacht wird:

a) mit Hilfe $H_2S$-sensitiven Elektrode,

b) mit Hilfe einer on-line-Sulfat-Bestimmungsanlage,

c) über eine Potentialbestimmung (Leitfähigkeitsmessung),

d) über eine Messung der freien $Me^{2+}$-Ionen-Konzentration.

24. Verfahren gemäss Anspruch 3, dadurch gekennzeichnet, dass man zur Ausfällung des toxischen $H_2S$ Metallsalze verwendet, die im sauren pH-Bereich löslich sind und, die im neutralen pH-Bereich zu einer Ausfällung in Form des entsprechenden Metallsulfids führen.

25. Verfahren gemäss Anspruch 3, dadurch gekennzeichnet, dass man zur Fällung des Metallsulfids ein Flockungsmittel zugibt.

26. Verfahren gemäss Anspruch 3, dadurch gekennzeichnet, dass die Abtrennung des ausgefällten Metallsulfids durch Sedimentation erfolgt.

27. Verfahren gemäss Anspruch 3, dadurch gekennzeichnet, dass man das anfallende $H_2S$-Gas nach dem Claus-Prozess zu elementarem Schwefels oxidiert.

28. Verfahren gemäss Anspruch 3, dadurch gekennzeichnet, dass man das anfallende $H_2S$-Gas nach Sconce und Berlinghoff zu Natrium-Sulfid- oder Natriumhydrogensulfidlösungen umsetzt.

29. Verfahren gemäss Anspruch 1 zum Recycling Schwermetall-haltiger Abfallschwefelsäure, dadurch gekennzeichnet, dass man anfallende Schwermetallsulfide mit verunreinigter Abfallschwefelsäure aufarbeitet.

30. Verfahren gemäss Anspruch 1, dadurch gekennzeichnet, dass die Sulfat-Reduktionsrate zwischen 15 g $SO_{4red}$/$\ell$ Bioreaktor/d und 60 g $SO_{4red}$/$\ell$ Bioreaktor/d liegt.